# EUROPEAN PATENT APPLICATION

(11) **EP 4 056 235 A1**
(43) Date of publication of application: **14.09.2022**
(21) Application number: 20884837.4
(22) Date of filing: 28.10.2020
(51) Int. Cl.: A61Q 17/04, A61K 8/19, A61K 8/894

(54) **OIL-IN-WATER COMPOSITION**

(30) Priority: 08.11.2019 JP 2019203590
(71) Applicant: Shiseido Company, Ltd., Chuo-ku Tokyo 104-0061 (JP)
(72) Inventor: SHARMA, Damini, Tokyo 104-0061 (JP); WATANABE, Yurika, Tokyo 104-0061 (JP)
(74) Representative: Ter Meer Steinmeister & Partner
(86) International application number: PCT/JP2020/040400
(87) International publication number: WO 2021/090740

(57) **Abstract**

An oil-in-water type composition in which powder is dispersed in an oil phase, the composition including: (A) 10% by mass or more of polar oil; (B) inorganic powder; (C) core-corona type microgel in which hydrophilic groups are partially provided on a surface of a hydrophobic fine particle; and (D) polyglycerin-modified silicone.

## Description

### [Technical Field]

The present invention relates to an oil-in-water type composition.

### [Background Art]

An oil-in-water type composition in which an oil phase is dispersed in a continuous aqueous phase can impart a pleasant physical sensation such as a fresh and moist physical sensation, and is therefore preferred and widely used as a topical composition, particularly a cosmetic. Many of the oil-in-water type compositions contain powder having an ultraviolet scattering function and the like. Typically, it is difficult to ensure stability of an oil-in-water type emulsion system containing such powder. Accordingly, a variety of studies have been conducted to improve stability of oil-in-water type compositions.

For example, PTL 1 discloses that adding an acrylic acid-derived polymer and an alkyl-modified water-soluble cellulose ether improves stability of an oil-in-water type composition containing powder.

### [Citation List]

### [Patent Literature]

[PTL 1]
Japanese Laid-Open Patent Publication No. 2012-140334

### [Summary of Invention]

### [Technical Problem]

The oil-in-water type composition containing powder as described above may require higher content of an oily component for some applications of the composition. However, such formulation may not provide sufficient stability of an oil-in-water type dosage form.

In view of the foregoing, it is an object of one aspect of the present invention to provide an oil-in-water type composition that exhibits good stability having a relatively high content of an oily component.

### [Solution to Problem]

In order to solve the above problem, one aspect of the present invention is: an oil-in-water type composition in which powder is dispersed in an oil phase, the composition including (A) 10% by mass or more of polar oil; (B) inorganic powder; (C) core-corona type microgel in which hydrophilic groups are partially provided on a surface of a hydrophobic fine particle; and (D) polyglycerin-modified silicone.

### [Advantageous Effects of Invention]

According to one aspect of the present invention, an oil-in-water type composition that exhibits good stability having a relatively high content of an oily component, can be provided.

### [Description of Embodiments]

Hereinafter, embodiments of the present invention will be described in detail. The present invention is not limited to the following embodiments.

An embodiment of the present invention is an oil-in-water type composition in which powder is dispersed in an oil phase, the composition including: (A) 10% by mass or more of polar oil; (B) inorganic powder; (C) core-corona type microgel in which hydrophilic groups are partially provided on a surface of a hydrophobic fine particle; and (D) polyglycerin-modified silicone. Thus, the composition according to the present embodiment is an oil-in-water type composition containing powder, and is also referred to as a powder in oil in water (POW) composition.

### <(A) polar oil>

The (A) polar oil is an oily component with a relatively high polarity that is commonly used in oil-in-water type compositions.

The content of the (A) polar oil in the composition of the present embodiment may be 10% by mass or more relative to the total amount of the oil-in-water type composition. Also, depending on the function or application of the composition, the content of the (A) polar oil may be preferably 15% by mass or more, more preferably 20% by mass or more, even more preferably 25% by mass or more, and even more preferably 30% by mass or more relative to the total amount of the composition. By setting the content of the (A) polar oil to 10% by mass or more, the stability of the oil-in-water type emulsion system containing powder is enhanced, and when the composition is used as a topical agent, moisturizing effect or what is known as occlusion effect (effect of blocking the skin surface to prevent water dissipation) can be enhanced. Also, according to the present embodiment, even when the composition contains a relatively high content of the (A) polar oil as in the range described above, or the composition contains a relatively high content of the (A) polar oil as in the range described above and further contains powder, the stability of the oil-in-water type dosage form of the composition can be improved.

Further, the content of the (A) polar oil may be preferably 55% by mass or less, more preferably 50% by mass or less, even more preferably 40% by mass or less, and even more preferably 35% by mass or less relative to the total amount of the composition. By setting the upper limit of the content of the (A) polar oil, it is possible to improve the stability of the dosage form, suppress unpleasant stickiness, and obtain a better usability.

The (A) polar oil may include an ultraviolet absorber. In other words, the (A) polar oil may include a polar oil having an ultraviolet absorbing function (Hereinafter, it may be referred to as an ultraviolet absorbing polar oil). When the (A) polar oil includes the ultraviolet absorber, the oil-in-water type composition according to the present embodiment can be suitably used as a cosmetic composition having a sunscreen property, or as a sunscreen composition.

The ultraviolet absorber (the ultraviolet absorbing polar oil) included in the (A) polar oil is not particularly limited. Examples of the ultraviolet absorber include benzoic acid derivatives, salicylic acid derivatives, cinnamic acid derivatives, dibenzoylmethane derivatives, β,β-diphenylacrylate derivatives, benzophenone derivatives, benzylidene camphor derivatives, phenylbenzimidazole derivatives, triazine derivatives, phenylbenzotriazole derivatives, anthranil derivatives, imidazoline derivatives, benzalmalonate derivatives, and 4,4-diarylbutadiene derivatives. More specific examples are listed below.

Examples of the benzoic acid derivatives include ethyl p-aminobenzoate (PABA), ethyl-dihydroxypropyl PABA, ethylhexyl-dimethyl PABA, glyceryl PABA, PEG-25-PABA, hexyl diethylaminohydroxybenzoyl benzoate, and the like.

Examples of the salicylic acid derivatives include homosalate, ethylhexylsalicylate (octyl salicylate), dipropylene glycol salicylate, TEA salicylate, and the like.

Examples of the cinnamic acid derivatives include octyl methoxycinnamate or ethylhexyl methoxycinnamate, isopropyl methoxycinnamate, isoamyl methoxycinnamate, cinoxate, DEA methoxycinnamate, diisopropyl methyl cinnamate, glyceryl-ethylhexanoate-dimethoxycinnamate, di-(2-ethylhexyl)-4'-methoxybenzalmalonate, and the like.

Examples of the dibenzoylmethane derivatives include butyl methoxydibenzoylmethane (4-tert-butyl-4'-methoxydibenzoylmethane) and the like. Examples of the β,β-diphenylacrylate derivatives include octocrylene and the like.

Examples of the benzophenone derivatives include benzophenone-1, benzophenone-2, benzophenone-3 or oxybenzone, benzophenone-4, benzophenone-5, benzophenone-6, benzophenone-8, benzophenone-9, benzophenone-12, and the like.

Examples of the benzylidene camphor derivatives include 3-benzylidene camphor, 4-methylbenzylidene camphor, benzylidene camphor sulfonic acid, camphor benzalkonium methosulfate, terephthalylidene dicamphor sulfonic acid, polyacrylamide methylbenzylidene camphor, and the like.

Examples of the phenylbenzimidazole derivatives include phenylbenzimidazole sulfonic acid, disodium phenyldibenzimidazole tetrasulfonate, and the like.

Examples of the triazine derivatives include anisotriazine (bis-ethylhexyloxyphenol methoxyphenyltriazine), ethylhexyltriazone, diethylhexylbutamide triazone, 2,4,6-tris(diisobutyl-4'-aminobenzalmalonate)-s-triazine, and the like.

Examples of the phenylbenzotriazole derivatives include drometrizole trisiloxane, methylenebis(benzotriazolyltetramethylbutylphenol), and the like.

Examples of the anthranil derivatives include menthyl anthranilate, and the like. Examples of the imidazoline derivatives include ethylhexyldimethoxybenzylidene dioxoimidazolidine propionate, and the like. Examples of the benzalmalonate derivatives include polyorganosiloxanes having benzalmalonate functional groups. Examples of the 4,4-diarylbutadiene derivatives include 1,1-dicarboxy(2,2'-dimethylpropyl)-4,4-diphenylbutadiene, and the like.

The ultraviolet absorbing polar oil described above may be used alone or in combination with two or more. It is preferable to use a combination of a plurality of ultraviolet absorbing polar oils including derivatives derived from different precursor compounds. Accordingly, a wide-range ultraviolet protection effect can be provided. For example, as the ultraviolet absorbing polar oil, a combination of diethylaminohydroxybenzoyl benzoate hexyl, homosalate, ethylhexylsalicylate (octyl salicylate), butylmethoxydibenzoylmethane, octocrylene, anisotriazine (bis-ethylhexyloxyphenol methoxyphenyltriazine), and ethylhexyltriazone can be used.

As described above, the ultraviolet absorbing polar oil is not particularly limited. In view of the environmental protection after disposal, it is preferable that the composition according to the present embodiment includes substantially no octyl methoxycinnamate, and it is more preferable that the composition does not include octyl methoxycinnamate at all.

As used herein, "include substantially no" or "contain substantially no" means that the content relative to the total amount of the composition or relative to the amount of a predetermined portion of the composition is preferably 0.1% by mass or less, more preferably 0.05% by mass or less, even more preferably 0.01% by mass or less, and even more preferably 0.001% by mass or less.

The content of the ultraviolet absorbing polar oil in the (A) polar oil is preferably 20% by mass or more, more preferably 30% by mass or more, even more preferably 40% by mass or more, even more preferably 60% by mass or more, even more preferably 70% by mass or more, and even more preferably 80% by mass or more relative to the total amount of the (A) polar oil.

The content of the ultraviolet absorbing polar oil relative to the total amount of the (A) polar oil may be 100% by mass. That is, in the composition according to the present embodiment, the (A) polar oil may be the ultraviolet absorbing polar oil. The content of the ultraviolet absorbing polar oil relative to the total amount of the (A) polar oil may be 98% by mass or less and 95% by mass or less.

The content of the ultraviolet absorbing polar oil relative to the total amount of the oil-in-water type composition is preferably 10% by mass or more, more preferably 20% by mass or more, and even more preferably 30% by mass or more. It is preferable that the content of the ultraviolet absorbing polar oil relative to the total amount of the oil-in-water type composition is 50% by mass or less, and more preferably 40% by mass or less. When the content of the ultraviolet absorbing polar oil is 10% by mass or more, the high ultraviolet protection function of the composition can be obtained. When the content of the ultraviolet absorbing polar oil is 50% by mass or more, it is possible to prevent the composition from becoming unpleasantly sticky when applied.

Thus, the (A) polar oil may contain only the ultraviolet absorbing polar oil, or may contain the ultraviolet absorbing polar oil and a polar oil having no ultraviolet absorbing function. As the polar oil having no ultraviolet absorbing function, ester oils are preferably used.

Examples of the ester oils include isononyl isononanoate, isopropyl myristate, cetyl 2-ethylhexanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxystearate, ethylene glycol di 2-ethylhexanoate, dipentaerythritol fatty acid ester, N-alkyl glycol monoisostearate, neopentyl glycol dicaprate, diisostearyl malate, glyceryl di 2-heptylundecanoate, trimethylol propane tri 2-ethylhexanoate, trimethylol propane triisostearate, pentaerythritol tetra-2-ethylhexanoate, glyceryl tri 2-ethylhexanoate (triethylhexanoin), trimethylolpropane triisostearate, cetyl 2-ethylhexanoate, 2-ethylhexylpalmitate, diethylhexyl naphthalene dicarboxylate, alkyl benzoate (12 to 15 carbons), cetearyl isononanoate, tri(caprylic acid/capric acid) glycerin, (dicaprylic acid/capric acid) butylene glycol, glyceryl trimyristate, tri 2-heptylundecanoate glyceride, castic oil fatty acid methyl ester, oleyl oleate, cetostearyl alcohol acetoglyceride, 2-heptylundecyl palmitate, diisobutyl adipate, 2-octyldodecyl N-lauroyl-L-glutamate, 2-heptylundecyl adipate, ethyllaurate, di 2-ethylhexyl sebacate, 2-hexyldecyl myristate, 2-hexyldecyl palmitate, 2-hexyldecyl adipate, diisopropyl sebacate, di 2-ethylhexyl succinate, ethyl acetate, butyl acetate, amyl acetate, triethyl citrate, 2-ethylhexyl p-methoxycinnamic acid, and tripropylene glycol dipivalate.

The ester oil described above may be used alone or in combination with two or more. When the composition contains the ester oil, dissolution of the solid ultraviolet absorber can be facilitated. Among the ester oils described above, it is preferable to use one or more of glyceryl tri 2-ethylhexanoate (triethylhexanoin), diisopropyl sebacate, and diethylhexyl succinate. It is more preferable to use glyceryl tri 2-ethylhexanoate (triethylhexanoin).

The content of the ester oil is preferably 0.5 to 20% by mass and more preferably 1 to 15% by mass relative to the total amount of the (A) polar oil. The content of the ester oil is preferably 0.1 to 8% by mass and more preferably 0.5 to 5% by mass relative to the total amount of the oil-in-water type composition.

The (A) polar oil preferably contains the ultraviolet absorbing polar oil and the ester oil.

From the viewpoint of solubility in the composition, it is preferable that the IOB value of the (A) polar oil is 0.1 to 0.5 and more preferably 0.1 to 0.4, regardless of the presence or absence of the ultraviolet absorbing function. The IOB value of the ultraviolet absorbing polar oil is preferably 0.1 to 0.3. The IOB value of the polar oil, particularly the ester oil, without ultraviolet absorbing function is preferably 0.05 to 0.8.

The IOB value is an abbreviation of the Inorganic/Organic Balance, which represents the ratio of the inorganic value to the organic value. The IOB value is an indicator of the degree of polarity of the organic compound. The IOB value is specifically expressed by an equation of "IOB value = inorganic value/organic value". The "inorganic value" and the "organic value" are determined according to atoms or functional groups. For example, the "organic value" is 20 for one carbon atom in a molecule, and the "inorganic value" is 100 for one hydroxyl group in the molecule. The IOB value of an organic compound can be calculated by summing up the "inorganic value" and the "organic value" of all atoms and functional groups in the compound (see, for example, Fujita, "Field of Chemistry", Volume 11, No. 10, pp. 719-725, 1957).

As is apparent from the examples of the (A) polar oil described above, the (A) polar oil may be solid or liquid at room temperature.

### <(B) inorganic powder>

(B) inorganic powder according to the present embodiment is not limited as long as the powder is capable of providing any function depending on the application of the composition such as ultraviolet scattering function, coloring function, aesthetic effect improvement function, and the like. The (B) inorganic powder may include those with a hydrophobic surface or a hydrophilic surface. The particles of the (B) inorganic powder may be spherical, needle-shaped, plate-shaped, or any other shape.

The (B) inorganic powder may contain an ultraviolet scattering agent. That is, the (B) inorganic powder may contain an inorganic powder having an ultraviolet scattering function. The average particle size of the primary particle of the inorganic powder having the ultraviolet scattering function is preferably 100 nm or less.

Examples of the inorganic powder having the ultraviolet scattering function include titanium oxide, zinc oxide, talc, silica, mica, sericite, kaolin, mica titanium, black iron oxide, yellow iron oxide, red iron oxide, ultramarine, iron blue, chromium oxide, chromium hydroxide, and the like. The inorganic powder may be composite powder in which particles other than titanium oxide are coated with titanium oxide. These inorganic powders may be used alone or in combination with two or more.

The inorganic powder having the ultraviolet scattering function is preferably inorganic powder in which the surface of powder particles is hydrophobic (it may be referred to as hydrophobic inorganic powder). The inorganic powder having the ultraviolet scattering function is preferably dispersed in the oil phase. The inorganic powder is preferably smaller than the particle size of the oil phase (a second oil phase, as described below) formed in the composition.

The hydrophobic inorganic powder may be obtained by introducing a hydrophobic group onto the particle surface of the inorganic powder, or by subjecting the particle surface of the inorganic powder a hydrophobic treatment to form a coating of hydrophobic material. The hydrophobic treatment may be a wet process using a solvent, a vapor phase process, a mechanochemical process, and the like. In the hydrophobic treatment, silicones such as methyl hydrogen polysiloxane, dimethyl polysiloxane, and the like, dextrin fatty acid esters, higher fatty acids, higher alcohols, fatty acid esters, lime soap, alkyl phosphate ethers, fluorine compounds, or hydrocarbons such as squalane, paraffin, and the like, may be used.

Among the powders described above, one or more of titanium oxide and zinc oxide is preferably used as the inorganic powder having the ultraviolet scattering function from the viewpoint that the ultraviolet scattering function is high. Particularly, hydrophobized titanium oxide is preferably used. It is known that titanium oxide tends to agglomerate in the composition depending on formulation of the oil-in-water type composition. According to the present embodiment, stability of the emulsion system can be obtained even when the titanium oxide is contained as the powder having the ultraviolet scattering function.

Preferably, the composition according to the present embodiment contains substantially no zinc oxide, or does not contain zinc oxide at all. Accordingly, the environmental impact at the time of disposal can be reduced. In a formulation containing butyl methoxybenzoylmethane as the ultraviolet absorbing polar oil, dispersibility of zinc oxide may be insufficient. By reducing the content of zinc oxide, the content of butyl methoxybenzoylmethane having a high ultraviolet absorbing function can be increased, thereby increasing the ultraviolet protection effect.

The (B) inorganic powder may include inorganic powder that does not have an ultraviolet scattering function, such as inorganic powder having an average particle size of 100 nm or more. The inorganic powder that does not have an ultraviolet scattering function may be, for example, inorganic powder capable of providing functions such as coloring, improving the aesthetics of the coated surface (giving gloss, covering uneven color), and the like.

Examples of the inorganic powder that does not have an ultraviolet scattering function include talc, kaolin, mica, silk mica (sericite), white mica, gold mica, synthetic mica, red mica, black mica, vermiculite, magnesium carbonate, calcium carbonate, aluminum silicate, barium silicate, calcium silicate, magnesium silicate, strontium silicate, metal tungstate salt, magnesium, silica, zeolite, barium sulfate, calcined calcium sulfate (baked calcium), calcium phosphate, fluorine apatite, hydroxyapatite, ceramic powder, lime soap (for example, zinc myristate, calcium palmitate, aluminum stearate), boron nitride, and the like.

The inorganic powder that does not have an ultraviolet scattering function may be a pigment. Examples of the pigment include inorganic white pigments (for example, titanium dioxide, zinc oxide, and the like), inorganic red pigments (for example, iron oxide (red iron oxide), iron titanate, and the like), inorganic brown pigments (for example, γ-iron oxide, and the like), inorganic yellow pigments (for example, yellow iron oxide, ocher, and the like), inorganic black pigments (for example, black iron oxide, low-order titanium oxide, and the like), inorganic violet pigments (for example, mango violet, cobalt violet, and the like), inorganic green pigments (for example, chromium oxide, chromium hydroxide, cobalt titanate, and the like), inorganic blue pigments (for example, ultramarine, iron blue, and the like), pearl pigments (for example, titanium oxide coated mica, titanium oxide coated bismuth, titanium oxide coated talc, colored titanium oxide coated mica, bismuth oxychloride, fish scale flake, and the like), and metal powder pigments (for example, aluminum powder, copper powder, and the like); organic pigments such as zirconium lake, barium lake, and aluminum lake; natural colorant; and the like.

The content of the (B) inorganic powder is preferably 0.1 to 20% by mass relative to the total amount of the oil-in-water type composition, more preferably 1 to 15% by mass, and even more preferably 3 to 10% by mass. According to the present embodiment, the stability of the dosage form of the oil-in-water type emulsion system can be improved even when the inorganic powder is contained in the amount described above. That is, the oil-in-water type composition can be provided in which high function of the inorganic powder is exhibited by containing a relatively high content of the inorganic powder and the stability is maintained.

The content of the inorganic powder having the ultraviolet scattering function is preferably 0.1 to 20% by mass, more preferably 1 to 15% by mass, and even more preferably 3 to 10% by mass, relative to the total amount of the oil-in-water type composition according to the present embodiment. By setting the content of the inorganic powder having the ultraviolet scattering function to the range described above, the composition can exhibit sufficient ultraviolet protection function while reducing the possibility that the stability of the oil-in-water type dosage form is deteriorated due to agglomeration of the powder.

The content of the inorganic powder that does not have an ultraviolet scattering function, such as silica, talc, and the like, is preferably 0.01 to 5% by mass relative to the total amount of the oil-in-water type composition according to the present embodiment. By setting the content of the inorganic powder that does not have the ultraviolet scattering function to the above range, it is possible to exhibit a predetermined function other than ultraviolet scattering by the inorganic powder while reducing the possibility that the stability of the oil-in-water type dosage form is deteriorated due to agglomeration of the powder.

### <(C) core-corona type microgel>

The (C) core-corona type microgel is a gel in which core-corona type microparticles are dispersed. The core-corona type microparticle has a lipophilic core part and a hydrophilic corona part. More specifically, the core-corona type microparticle is a hydrophobic fine particle in which hydrophilic groups are partially provided on the surface. The (C) core-corona type microgel is used as an emulsifier to form and maintain well the oil-in-water type emulsion. By using the (C) core-corona type microgel as an emulsifier in the oil-in-water type composition that contains powder in the oil phase, the fresh and moist physical sensation can be improved, and the water resistance of the composition can be improved.

The core-corona type microparticle may be either crosslinked or non-crosslinked. Suitable crosslinked core-corona type microparticles include an (acrylates/methoxy PEG methacrylates) crosspolymer. Suitable non-crosslinked cores-corona type microparticles include an (acrylamides/DMAPA acrylates/methoxy PEG methacrylates) copolymer.

### 1. Crosslinked core-corona type microparticle

The crosslinked core-corona type microparticle may be obtained by radical polymerization of the monomers represented by formulae (1) to (3) described below under specific conditions. Specific examples include a (acrylates/methoxy PEG-90 methacrylate) crosspolymer.

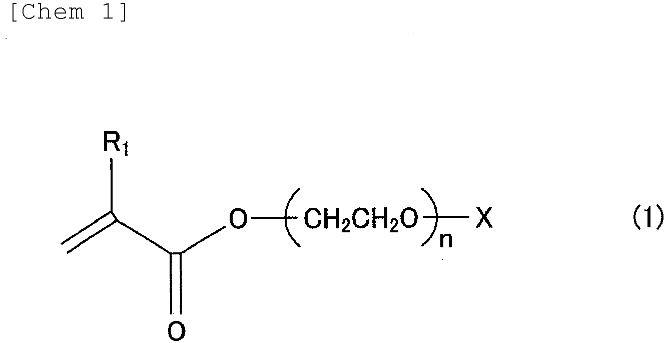

In formula (1), R1 is an alkyl group of 1 to 3 carbons, and n is a number of 8 to 200. X is H or CH₃.

Examples of the polyethylene oxide macromonomer represented by formula (1) described above include commercially available products supplied by Sigma-Aldrich Co. LLC, BLEMMER (registered trademark) PME-400 and BLEMMER (registered trademark) PME-1000, and BLEMMER (registered trademark) PME-4000 manufactured by NOF CORPORATION, and the like.

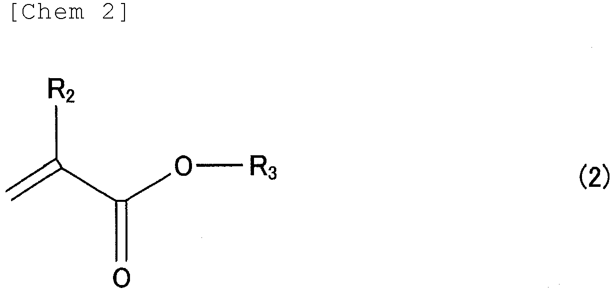

In formula (2), R2 is an alkyl group of 1 to 3 carbons. R3 may be an alkyl group of 1 to 12 carbons, preferably an alkyl group of 1 to 8 carbons.

As the hydrophobic monomer represented by formula (2) described above, for example, commercially available products supplied by Sigma-Aldrich Co. LLC and Tokyo Chemical Industry Co., Ltd. may be used.

Examples of the hydrophobic monomer include methyl acrylate, ethyl acrylate, propyl acrylate, butyl acrylate, pentyl acrylate, hexyl acrylate, heptyl acrylate, octyl acrylate, decyl acrylate, dodecyl acrylate, methyl methacrylate, ethyl methacrylate, propyl methacrylate, butyl methacrylate, pentyl methacrylate, hexyl methacrylate, heptyl methacrylate, octyl methacrylate, decyl methacrylate, dodecyl methacrylate, and the like. In particular, methyl methacrylate, butyl methacrylate, and octyl methacrylate are preferably used.

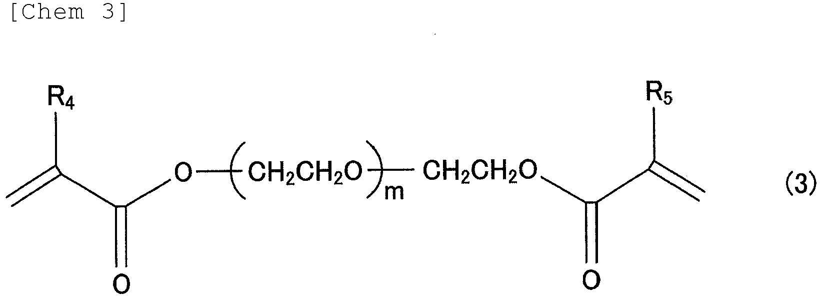

In formula (3), R4 and R5 independently represent an alkyl group of 1 to 3 carbons, and m is a number of 0 to 2.

The crosslinkable monomer represented by formula (3) described above is preferably hydrophobic. Examples of the crosslinkable monomer include, preferably, ethylene glycol dimethacrylate (hereinafter, it may be referred to as EGDMA) supplied by Sigma-Aldrich Co. LLC, BLEMMER (registered trademark) PDE-50 manufactured by NOF CORPORATION, and the like.

The core-corona type microparticle is obtained by radical polymerization of the monomers described above under the conditions (1a) to (1e) below:
(1a) the ratio of the prepared molar quantity of the polyethylene oxide macromonomer to the prepared molar quantity of the hydrophobic monomer (the amount of the macromonomer : the amount of the hydrophobic monomer) is 1:10 to 1:250;
(1b) the prepared amount of the crosslinkable monomer is 0.1 to 1.5% by mass relative to the prepared amount of the hydrophobic monomer;
(1c) the hydrophobic monomer is a mixture of one or more monomers of the methacrylic acid derivative represented by formula (2) having an alkyl group of 1 to 8 carbons;
(1d) a polymerization solvent is a mixed solvent of water and organic solvent, and when polyol is used as the organic solvent, the polyol is one or more selected from dipropylene glycol, 1,3-butylene glycol, and isoprene glycol; and
(1e) the ratio of the mass of the water to the mass of the organic solvent (the amount of the water : the amount of the organic solvent) of the mixed solvent of water and organic solvent at 20°C is 90:10 to 10:90.

### 2. Non-crosslinked core-corona type microparticle

The non-crosslinked core corona microparticle may be obtained by radical polymerization of the monomers represented by formulae (1), (2), and (4) described below under specific conditions. Specific examples include an (acrylamides/DMAPA acrylates/methoxy PEG methacrylates) copolymer.

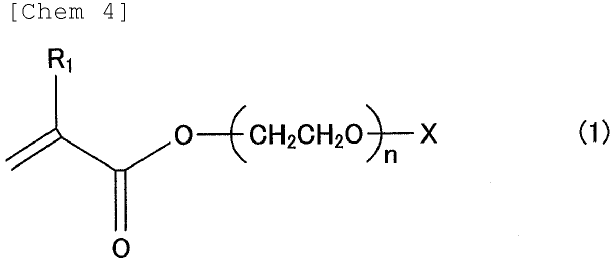

In formula (1), R1 is an alkyl group of 1 to 3 carbons, and n (number of polyethylene oxide moieties) is a number of 8 to 200. X is H or CH₃.

The polyethylene oxide macromonomer represented by formula (1) described above is preferably acrylic acid derivatives or methacrylic acid derivatives. For example, the polyethylene oxide macromonomer may be commercially available products supplied by Sigma-Aldrich Co. LLC, or BLEMMER series manufactured by NOF CORPORATION such as PME-400, PME-1000, and PME-4000 (values of n in the formula (1) are n=9, n=23, and n=90, respectively), and the like, which are methoxypolyethylene glycol monomethacrylates.

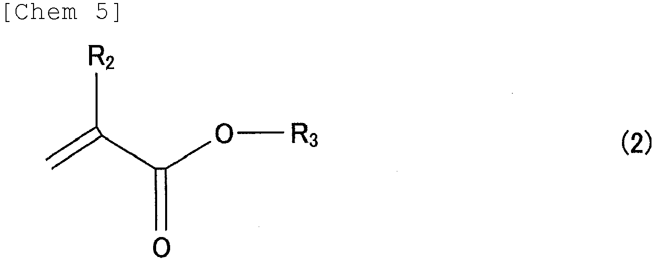

In formula (2), R2 represents an alkyl group of 1 to 3 carbons, and R3 represents a substituent containing an alkyl group of 1 to 12 carbons.

The hydrophobic monomer represented by formula (2) described above is preferably acrylic acid derivatives or methacrylic acid derivatives. Specific examples of the hydrophobic monomer include methyl acrylate, ethyl acrylate, propyl acrylate, butyl acrylate, pentyl acrylate, hexyl acrylate, heptyl acrylate, octyl acrylate, decyl acrylate, dodecyl acrylate, methyl methacrylate, ethyl methacrylate, propyl methacrylate, butyl methacrylate, pentyl methacrylate, hexyl methacrylate, heptyl methacrylate, octyl methacrylate, decyl methacrylate, dodecyl methacrylate, and the like. In particular, methyl methacrylate, butyl methacrylate, and octyl methacrylate are preferably used.

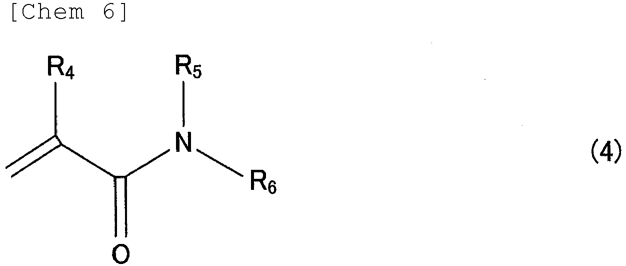

In formula (4), R4 represents H or an alkyl group of 1 to 3 carbons, R5 and R6 independently represent H or a substituent containing an alkyl group of 1 to 18 carbons.

The hydrophobic monomer represented by formula (4) described above is preferably acrylamide derivatives or methacrylamide derivatives. Specific examples of the hydrophobic monomer include t-butyl acrylamide, N,N-dimethyl acrylamide, N-[3-(dimethylamino)propyl]acrylamide, t-butyl methacrylamide, octyl acrylamide, octyl methacrylamide, octadecyl acrylamide, and the like. Among these, t-butylacrylamide, N,N-dimethyl acrylamide, and N-[3-(dimethylamino)propyl]acrylamide are preferred.

The copolymer constituting the core-corona type microparticle is obtained by copolymerizing the macromonomer represented by formula (1) described above and one or more of the hydrophobic monomers represented by the formulae (2) and (4) described above using any radical polymerization method under the conditions (2a) to (2d) below:
(2a) the ratio of the prepared molar quantity of the polyethylene oxide macromonomer to the prepared molar quantity of the acrylate derivative monomer and/or the acrylamide derivative monomer (the amount of the macromonomer : the amount of the derivative monomer) is 1:10 to 1:250;
(2b) the macromonomer represented by formula (1) described above is an acrylic acid derivative or a methacrylic acid derivative having a polyethylene glycol group having a repeating unit of 8 to 200;
the acrylate derivative monomer represented by formula (2) described above is an acrylic acid derivative or a methacrylic acid derivative having a substituent containing an alkyl group of 1 to 12 carbons; and the acrylamide derivative monomer represented by formula (4) described above is an acrylamide derivative or a methacrylamide derivative having a substituent containing an alkyl group of 1 to 18 carbons;
(2c) a polymerization solvent is a mixed solvent of water and alcohol, and the alcohol is one or more selected from ethanol, dipropylene glycol, 1,3-butylene glycol, and isoprene glycol; and
(2d) the ratio of the mass of the water to the mass of the alcohol (the amount of the water : the amount of the alcohol) of the mixed solvent of water and alcohol at 20°C is 90:10 to 10:90.

The content of the core-corona type microparticle is preferably 0.01 to 10% by mass, preferably 0.05 to 5% by mass, and more preferably 0.1 to 3% by mass relative to the total amount of the oil-in-water type composition. When the content of the core-corona type microparticle is set to 0.01% by mass or more, it is possible to stabilize the oil phase-in-water type dosage form in which the oil phase (inner phase) is dispersed in the aqueous phase (outer phase). When the content is set to 10% by mass or less, the long-term storage stability under high temperature conditions can be improved.

When preparing the composition according to the present embodiment, the core-corona type microparticle may be dispersed in a solvent containing water and/or alcohol to prepare a dispersion.

### (D) polyglycerin-modified silicone

The (D) polyglycerin-modified silicone according to the present embodiment is a compound having a glycerin chain part and a silicone chain part in combination. By using the (D) polyglycerin-modified silicone, because the glycerin chain part is well adsorbed to the inorganic powder and the silicone chain part is well dissolved in the oily component, the inorganic powder can be well dispersed in the oil phase.

The (D) polyglycerin-modified silicone may be a silicone in which glycerin chains are introduced. The (D) polyglycerin-modified silicone is preferably glycerin or polyglycerin in which both ends are modified by silicone (it may be referred to as both-end silicone modified glycerin or both-end silicone modified polyglycerin).

The (D) polyglycerin-modified silicone may be both-end silicone modified glycerin represented by structural formula (5), preferably structural formula (6) described below.

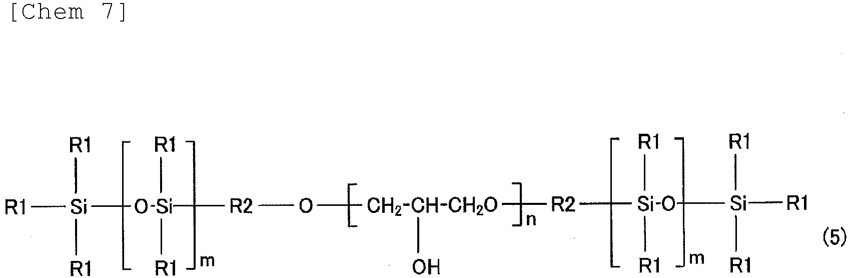

In the formula described above, R1 is a straight or branched alkyl group of 1 to 12 carbons or a phenyl group, R2 is an alkyl group of 2 to 11 carbons, m is 10 to 120, and n is 1 to 11.

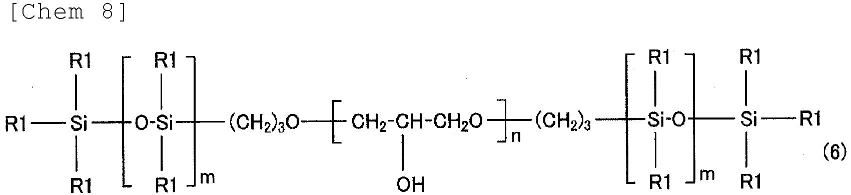

In the formula described above, R1 is a straight or branched alkyl group of 1 to 12 carbons or a phenyl group, m is 10 to 120, and n is 1 to 11.

The hydrophilic-lipophilic balance (HLB) of the (D) polyglycerin-modified silicone is preferably 0.2 to 3.0. The HLB value may be calculated using Griffin's expression (HLB value = glycerin part molecular weight × 20/total molecular weight). The molecular weight of the (D) polyglycerin-modified silicone is preferably 2,000 to 20,000.

Examples of the (D) polyglycerin-modified silicone include polyglyceryl-3 disiloxane dimethicone, polyglyceryl-3 polydimethylsiloxyethyl dimethicone, lauryl polyglyceryl-3 polydimethylsiloxyethyl dimethicone, bis-butyldimethicone polyglyceryl-3, a (dimethicone/polyglycerin-3) crosspolymer, a (lauryl dimethicone/polyglycerin-3) crosspolymer, a (polyglyceryl-3/lauryl polydimethylsiloxyethyl dimethicone) crosspolymer, and the like. These (D) polyglycerin-modified silicones may be used alone or in combination with two or more.

Examples of commercially available products of the polyglycerin-modified silicone described above include KF-6100, KF-6104, KF-6105, KF-6106, KF-6109, KSG-710, KSG-810, KSG-820, KSG-830, KSG-840, KSG-820Z, and KSG-850Z manufactured by Shin-Etsu Chemical Co., Ltd.

The (D) polyglycerin-modified silicone used in the present embodiment is preferably the non-crosslinked polyglycerin-modified silicone that does not constitute a crosspolymer, and more preferably one or more of polyglyceryl-3 disiloxane dimethicone, polyglyceryl-3 polydimethylsiloxyethyl dimethicone, lauryl polyglyceryl-3 polydimethylsiloxyethyl dimethicone, and bis-butyldimethicone polyglyceryl-3. Because the function of dispersing the inorganic powder in the oil phase is particularly high in the oil-in-water type composition, bis-butyldimethicone polyglyceryl-3 is further preferred.

The content of the (D) polyglycerin-modified silicone is preferably 0.05 to 5% by mass, more preferably 0.1 to 3% by mass, and even more preferably 0.3 to 2.5% by mass relative to the total amount of the oil-in-water type composition. When the content of the (D) polyglycerin-modified silicone is 0.05% by mass or more, the dispersibility of the (B) inorganic powder, particularly the inorganic powder having a relatively small particle size, can be improved. When the content is 5% by mass or less, the stability of the oil-in-water emulsion system containing the powder can be well maintained.

When bis-butyldimethicone polyglyceryl-3 is used, the content of bis-butyldimethicone polyglyceryl-3 is preferably 20 to 100% by mass, more preferably 30 to 95% by mass, and even more preferably 40 to 90% by mass relative to the total amount of the (D) polyglycerin-modified silicone.

The ratio of the content of the (D) polyglycerin-modified silicone to the content of the (B) inorganic powder described above is preferably 0.01 to 2, and more preferably 0.05 to 1.

### <Other component>

The oil-in-water type composition according to the present embodiment may include other optional components in addition to the components (A) to (D) described above. The other components include an oily component other than the (A) polar oil, an aqueous component, a surfactant, a transdermal absorption inhibitor, a chelating agent, an oil gelling agent, and the like. These components may be added in an amount that does not interfere with the effect of improving the stability of the oil-in-water type dosage form obtained by the present embodiment.

The oily component other than the (D) polar oil is not particularly limited and may be an oily component commonly used in topical compositions such as cosmetics, quasi-drugs, and the like. Examples of the oily component other than the polar oil include hydrocarbon oils, higher fatty acids, higher alcohols, silicone oils, liquid oils, solid fats, waxes, flavorings, and the like. As the other component, an ultraviolet absorber that is not the polar oil may be included.

Examples of the hydrocarbon oils include isododecane, isohexadecane, isoparaffin, liquid paraffin, ozokerite, squalane, pristane, paraffin, ceresin, squalene, petrolatum, microcrystalline wax, and the like. Among these, isododecane is preferable because the powder, particularly titanium oxide, can be dispersed well and the tactile sensation can be improved.

Examples of the higher fatty acids include lauric acid, myristic acid, palmitic acid, stearic acid, behenic acid, oleic acid, undecylenic acid, tall acid, isostearic acid, linoleic acid, linolenic acid, eicosapentaenoic acid (EPA), docosahexaenoic acid (DHA), and the like.

Examples of the higher alcohols include straight-chain alcohols (for example, lauryl alcohol, cetyl alcohol, stearyl alcohol, behenyl alcohol, myristyl alcohol, oleyl alcohol, cetostearyl alcohol, and the like), and branched-chain alcohols (for example, monostearyl glycerin ether (batyl alcohol)-2-decyltetradecynol, lanolin alcohol, cholesterol, phytosterol, hexyldodecanol, isostearyl alcohol, octyldodecanol, and the like), and the like.

Examples of the silicone oils include chain polysiloxanes (for example, dimethyl polysiloxane, methylphenyl polysiloxane, diphenyl polysiloxane, and the like), cyclic polysiloxanes (for example, octamethylcyclotetrasiloxane, decamethylcyclopentasiloxane, dodecamethylcyclohexasiloxane, and the like), a silicone resin forming a three-dimensional network structure, a silicone rubber, modified polysiloxanes (for example, amino modified polysiloxane, polyether modified polysiloxane, alkyl modified polysiloxane, fluorine modified polysiloxane, and the like), acrylic silicones, and the like. Among these, chain polysiloxanes such as dimethyl polysiloxane is preferable because a good tactile sensation can be obtained. The composition according to the present embodiment preferably contains substantially no cyclic polysiloxanes, or does not contain cyclic polysiloxanes at all, in consideration of the environment at the time of disposal.

The aqueous component is not particularly limited, and may be an aqueous component commonly used in topical compositions such as cosmetics, quasi-drugs, and the like. As the aqueous component, water, water-soluble alcohol, a thickening agent, a humectant, a chelating agent, a preservative, colorant, and the like may be used.

As the water, purified water, ion-exchanged water, tap water, and the like may be used. In the present embodiment, the content of the water is preferably 20 to 70% by mass, and more preferably 30 to 60% by mass relative to the total amount of the oil-in-water type composition.

Examples of the water-soluble alcohol include lower alcohol, polyhydric alcohol, polyhydric alcohol polymer, alcohol alkyl ether, alcohol ether ester, glycerin monoalkyl ether, sugar alcohol, monosaccharide, oligosaccharide, and polysaccharide, and derivatives thereof, and the like.

Examples of the lower alcohol include ethanol, propanol, isopropanol, isobutyl alcohol, t-butyl alcohol, and the like.

Examples of the polyhydric alcohol include dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, octylene glycol, and the like); trihydric alcohols (for example, glycerin, trimethylol propane, and the like); tetrahydric alcohols (for example, diglycerine, 1,2,6-hexanetriol, pentaerythritol, and the like); pentahydric alcohols (for example, xylitol, triglycerin, and the like); hexahydric alcohols (for example, sorbitol, mannitol and the like), and the like.

Examples of the thickening agent include plant polymers (for example, gum arabic, tragant gum, galactan, guar gum, carrageenan, and the like); microbial polymers (for example, xanthan gum, dextran, succinoglycan, pullulan, and the like); animal polymers (for example, collagen, casein, albumin, gelatin, and the like), starch polymers (for example, carboxymethyl starch, methyl hydroxypropyl starch, and the like); alginate polymers (for example, sodium alginate, propylene glycol alginate, and the like), and the like. The thickening agent may be a synthetic crosspolymer such as dimethyl acrylamide/acryloyl dimethyl taurine sodium crosspolymer.

The surfactant may be a cationic surfactant, an anionic surfactant, a nonionic surfactant, an amphoteric surfactant, and the like. Among these, a nonionic surfactant may be preferably used. The surfactant component acts as an emulsifier, and can improve the stability of the dosage form of the oil-in-water type emulsion.

The nonionic surfactant is not particularly limited, and may be a nonionic surfactant commonly used in a topical agent such as a cosmetic and a quasi-drug. More specific examples of the nonionic surfactant include polyoxyethylene hydrogenated castor oil such as PEG-40 hydrogenated castor oil, PEG-60 hydrogenated castor oil, PEG-100 hydrogenated castor oil, and the like; polyoxyethylene/methyl polysiloxane copolymers such as PEG-3 dimethicone, PEG-9 dimethicone, and the like; polyglycerin fatty acid esters such as polyglyceryl-2 diisostearate, polyglyceryl-2 triisostearate, and the like; monoglycerin fatty acid esters such as glyceryl stearate, glyceryl oleate, and the like; polyoxyethylene sorbitan fatty acid esters such as polyoxyethylene sorbitan monooleate, polyoxyethylene sorbitan monostearate, and the like; sorbitan fatty acid esters such as sorbitan sesquioleate, sorbitan sesquiisostearate, sorbitan isostearate, sorbitan palmitate, and the like; self-emulsifying propylene glycol stearate; and the like.

The content of the surfactant is preferably 0.5 to 10% by mass, and more preferably 1 to 5% by mass relative to the total amount of the oil-in-water type composition. The content of the nonionic surfactant is preferably 0.5 to 10% by mass, and more preferably 1 to 5% by mass relative to the total amount of the oil-in-water type composition.

Examples of the other component further include an antioxidant, a preservative, an antiinflammatory agent, a whitening agent, a plant extract, an activator, a blood circulation promoter, an anti-seborrheic agent, and the like. These components may be added in an amount that does not interfere with the effect of improving the stability of the dosage form of the oil-in-water type emulsion obtained by the present embodiment.

The composition according to the present embodiment may contain organic powder including resin powder such as polyamide and polyethylene. The composition according to the present embodiment preferably contains substantially no organic powder, or does not contain organic powder at all, in consideration of the environment at the time of disposal.

In the present embodiment, the oil-in-water type composition may be formed in which two different oil phases are dispersed in a continuous aqueous phase. For example, the composition may include a first oil phase mainly including the (A) polar oil and a second oil phase mainly including the (B) inorganic powder. More specifically, the first oil phase may mainly include the ultraviolet absorbing polar oil, and the second oil phase may mainly include the inorganic powder having an ultraviolet scattering function.

The second oil phase may be a phase in which the inorganic powder having an ultraviolet scattering function is dispersed in the oily component. The oily component for dispersing the inorganic powder in the second oil phase may be the (A) polar oil that does not have an ultraviolet absorbing function described above, or the oily component other than the (A) polar oil described above. It is preferable that the (D) polyglycerin-modified silicone is contained more in the second oil phase than in the first oil phase.

The oil-in-water type composition in which two different oil phases are dispersed in an aqueous phase may be obtained by respectively mixing the components constituting the first oil phase and the components constituting the second oil phase to give two mixtures, and then mixing the mixtures with the aqueous component and optionally with the other oily component.

The application of the oil-in-water type composition according to the present embodiment is not particularly limited. The composition is preferably used as a topical composition, particularly as a cosmetic composition. The oil-in-water type composition according to the present embodiment is also suitably used as a cosmetic composition having an ultraviolet absorbing function. For example, the composition may be a sunscreen composition and may be a cosmetic having an enhanced ultraviolet absorbing function, for example, basic cosmetics such as foundation and base make-up, make-up cosmetics, and the like.

According to the formulation of the present embodiment, a sunscreen composition having a higher content of an ultraviolet absorber and an ultraviolet scattering agent and having a higher ultraviolet protection effect can be provided. More specifically, a sunscreen composition having Sun Protection Factor (SPF) of 30 or more, preferably 40 or more, and more preferably 50 or more, can be provided. Also, a sunscreen composition having Protection grade of UVA (PA) of more than 4, and preferably more than 8, can be provided.

### [Example]

The components described in Table 1 were mixed by a conventional method to prepare the compositions of Example 1 to Example 5, and then the compositions were evaluated. Table 1 also describes the evaluation results.

### <Evaluation of emulsification and stability>

The compositions of each example were evaluated in terms of emulsification during preparation and stability of emulsion system after a predetermined time. The evaluation was performed visually based on the following criteria.
A: Emulsification was observed with good stability. Stability was maintained after one month.
B: Emulsification was observed with good stability. Oily component became separated after 24 hours.
C: Emulsification was observed but pahse inversion occured.
D: No emulsification was observed.

**[Table 1]**

| | | EXAMPLE 1 | EXAMPLE 2 | EXAMPLE 3 | EXAMPLE 4 | EXAMPLE 5 | EXAMPLE 6 |
|---|---|---|---|---|---|---|---|
| AQUEOUS COMPONENT | ION-EXCHANGED WATER | 31.47% | 29.97% | 30.97% | 30.17% | 36.47% | 31.97% |
| | ETHANOL (INDUSTRIAL ALCOHOL 95 DEGREES) | 5.0% | 5.0% | 5.0% | 5.0% | 5.0% | 5.0% |
| | GLYCERIN | 1.0% | 1.0% | 1.0% | 1.0% | 1.0% | 1.0% |
| | DIPROPYLENE GLYCOL | 3.0% | 3.0% | 3.0% | 3.0% | 3.0% | 3.0% |
| THICKENING AGENT | DIMETHYL ACRYLAMIDE/ACRYLOYL DIMETHYL TAURINE SODIUM CROSSPOLYMER | 0.5% | 0.5% | 0.5% | 0.5% | 0.5% | 0.5% |
| | SUCCINOGLYCAN | 02% | 0.2% | 0.2% | 0.2% | 02% | 02% |
| EMULSIFIER | ACRYLAMIDES/DMAPA ACRYLATES/METHOXY PEG METHACRYLATES COPOLYMER | 7.0% | 7.0% | 7.0% | 7.0% | - | 7.0% |
| | HYDROPHOBIZED HYDROXYPROPYLMETHYL CELLULOSE | 0.08% | 0.08% | 0.08% | 0.08% | 0.08% | 0.08% |
| DISPERSANT | LAURYL POLYGLYCERYL-3 POLYDIMETHYLSILOXYETHYL DIMETHICONE | - | - | 0.5% | - | - | - |
| | BIS-BUTYLDIMETHICONE POLYGLYCERYL-3 | 0.5% | 2.0% | 0.5% | 1.0% | 1.0% | - |
| | ISOSTEARIC ACID | - | - | - | 0.8% | 0.8% | - |
| SURFACTANT | SORBITAN SESQUIOLEATE | 0.2% | 0.2% | 0.2% | 0.2% | 02% | 02% |
| | PEG-100 HYDROGENATED CASTOR OIL | 1.0% | 1.0% | 1.0% | 1.0% | 1.0% | 1.0% |
| | PEG-60 HYDROGENATED CASTOR OIL | 2.0% | 2.0% | 2.0% | 2.0% | 2.7% | 2.0% |
| OILY COMPONENT | DIMETHICONE | 1.0% | 1.0% | 1.0% | 1.0% | 1.0% | 1.0% |
| TRANSDERMAL ABSORPTION INHIBITOR | PPG1000 | 1.0% | 1.0% | 1.0% | 1.0% | 1.0% | 1.0% |
| INORGANIC POWDER DISPERSION SYSTEM | ISODODECANE | 8.0% | 8.0% | 8.0% | 8.0% | 8.0% | 8.0% |
| | TRIETHYLHEXANOIN | 2.0% | 2.0% | 2.0% | 2.0% | 2.0% | 2.0% |
| | HYDROPHOBIZED TITANIUM OXIDE | 5.0% | 5.0% | 5.0% | 5.0% | 5.0% | 5.0% |
| OILY COMPONENT (POLAR OIL HAVING ULTRAVIOLET ABSORBING FUNCTION) | HOMOSALATE | 10.0% | 10.0% | 10.0% | 10.0% | 10.0% | 10.0% |
| | OCTOCRYLENE | 5.0% | 5.0% | 5.0% | 5.0% | 5.0% | 5.0% |
| | ETHYLHEXYLSALICYLATE | 5.0% | 5.0% | 5.0% | 5.0% | 5.0% | 5.0% |
| | ETHYLHEXYLTRIAZONE | 3.0% | 3.0% | 3.0% | 3.0% | 3.0% | 3.0% |
| | BUTYL METHOXYDIBENZOYLMETHANE | 2.3% | 2.3% | 2.3% | 2.3% | 2.3% | 2.3% |
| | BIS-ETHYLHEXYLOXYPHENOL METHOXYPHENYLTRIAZINE | 3.0% | 3.0% | 3.0% | 3.0% | 3.0% | 3.0% |
| | HEXYL DIETHYLAMINOHYDROXYBENZOYL BENZOATE | 2.2% | 2.2% | 2.2% | 2.2% | 2.2% | 2.2% |
| OIL GELLING AGENT | DEXTRIN PALMITATE | 0.5% | 0.5% | 0.5% | 0.5% | 0.5% | 0.5% |
| CHELATING AGENT | EDTA TETRASODIUM SALT | 0.05% | 0.05% | 0.05% | 0.05% | 0.05% | 0.05% |
| | TOTAL AMOUNT | 100% | 100% | 100% | 100% | 100% | 100% |
| | AMOUNT OF POLAR OIL | 32.5% | 32.5% | 32.5% | 32.5% | 32.5% | 32.5% |
| EVALUATION | EMULSIFICATION AND STABILITY | A | A | A | A | D | C |

The percentages in Table 1 indicate % by mass.

As illustrated in Table 1, Examples 1 to 4 which include (A) 10% by mass or more of polar oil, (B) inorganic powder, (C) core-corona type microgel, and (D) polyglycerin-modified silicone exhibited excellent emulsification with good emulsion system stability. In contrast, Example 5 which does not include (C) core-corona type microgel, and Example 6 which does not include (D) polyglycerin-modified silicone exhibited poor emulsification.

The present application claims priority to Japanese Patent Application No. 2019-203590, filed November 8, 2019, with the Japanese Patent Office. The contents of which are incorporated herein by reference in their entirety.

## Claims

1. An oil-in-water type composition in which powder is dispersed in an oil phase, the composition comprising:
(A) 10% by mass or more of polar oil;
(B) inorganic powder;
(C) core-corona type microgel in which hydrophilic groups are partially provided on a surface of a hydrophobic fine particle; and
(D) polyglycerin-modified silicone.

2. The composition according to claim 1, wherein the (A) polar oil includes an ultraviolet absorber.

3. The composition according to claim 2, wherein an amount of the ultraviolet absorber in the (A) polar oil is 20% by mass or more relative to 100% by mass of the (A) polar oil.

4. The composition according to any one of claims 1 to 3, wherein the (B) inorganic powder includes an ultraviolet scattering agent.

5. The composition according to any one of claims 1 to 4, wherein the (D) polyglycerin-modified silicone includes a both-end silicone modified polyglycerin.

6. The composition according to any one of claims 1 to 5, wherein SPF is 30 or more.

7. The composition according to any one of claims 1 to 6, wherein the composition is a cosmetic composition.

8. The composition according to any one of claims 1 to 7, wherein the composition is a sunscreen composition.
